Europäisches Patentamt

European Patent Office

Office européen des brevets

(11) Publication number: **0 265 100**
**A2**

# EUROPEAN PATENT APPLICATION

(21) Application number: 87308719.1

(22) Date of filing: 01.10.87

(51) Int. Cl.4: **A01H 1/02** , C12N 15/00

(30) Priority: 01.10.86 US 913913

(43) Date of publication of application:
27.04.88 Bulletin 88/17

(84) Designated Contracting States:
AT BE CH DE ES FR GB GR IT LI LU NL SE

(71) Applicant: THE PLANT CELL RESEARCH
INSTITUTE INC.
6560 Trinity Court
Dublin California 94568(US)

(72) Inventor: Trulson, Anna J.
7000 Tesla Road
Livermore California 94550(US)
Inventor: Shaheen, Elias A.
2929 Trotter Way
Walnut Creek California 94596(US)

(74) Representative: Goldin, Douglas Michael et al
J.A. KEMP & CO. 14, South Square Gray's Inn
London WC1R 5EU(GB)

(54) **Methods for controlled regeneration of cucumis sp plants in vitro from protoplasts.**

(57) Generation of competent <u>Cucumis</u> sp. embryoids, plantlets and plants (including seeds) from protoplasts, e.g., from cotyledonary , root, leaf or stem tissue, by isolation in enzyme solution followed by cell wall formation, formation of multicellular colonies, formation of minicalli with induction of embryoids, plating of minicalli with maturation of embryoids, and plantlet development from the embryoids in a series of media, CPM-1, -2 and -3, and CTM-4, containing two or more of NAA, ZR, 2,4-D, BAP and $GA_3$ under defined temperature, time and light conditions. Serial transfer to novel enzyme and <u>Cucumis</u> protoplast induction and expression media for defined periods results in cell wall formation, cell division, competent minicalli formation, and induction and expression (maturation) of competent embryoids and plantlets. Transfer to hormone-free development media permits plantlet development, which in turn progress to mature normal, flowering, fruiting plants. The process is applicable to producing encapsulated embryoids from single genotypes or from somatic hybrids as "artificial seeds". The process applied to increase of F-1 hybrids permits production of "artificial seeds" (encapsulations) in 1/2 to 1/10 the time it would take to produce natural hybrid seeds. Techniques involving $p_x/c_y$ somatic hybridization, freeze storage, and post-thawing development are disclosed. Sexual sterility barriers between plants in the genus <u>Cucumis</u> preventing genetic transfer of important characteristics are overcome.

## METHODS FOR CONTROLLED REGENERATION OF CUCUMIS SP PLANTS IN VITRO FROM PROTOPLASTS

The plants regenerated by the methods and media of this case may provide explant tissue to be further regenerated by the explant regeneration techniques of our copending application (our reference N 43003, hereinafter referred to as 92/9) or may be genetically transformed with Agrobacterium rhizogenes as set forth in our copending application (our reference N 43005, hereinafter referred to as 92/12), both filed of even date herewith. Likewise, the somatic hybrids produced by the techniques of this case may be regenerated or subjected to somaclonal variant screening and selection as set forth in No 92/9 or transformed as in 92/12. The disclosures of those cases are hereby incorporated by reference herein to the extent needed.

### Field

The present invention relates to controlled regeneration of Cucumis sp. plants in vitro. More specifically, this invention relates to methods of regeneration of embryoids, plantlets and plants from protoplasts liberated from plant tissue, whether wild or cultivated, hybrid (sexual or somatic), or genetically variant or transformed plants. The embryoids induced from the protoplasts are fully capable of completing development into mature plants, by use of solid (agar) or liquid cell suspension culture techniques employing a novel series of media for induction and maturation of the embryoids. Serial subculturing permits relatively continuous production of secondary, tertiary, etc. embryoids which can be stored frozen or encapsulated as "artificial seeds". This is particularly advantageous in the case of hybridization, where use of protoplasts from hybrid explant tissue from a natural F-1 hybrid or from somatic hybrid callus can produce such embryoids for encapsulation as artificial seeds in a much shorter time than required for the full cycle of plant maturation and seed development.

### Background

Sterility barriers between species are among the most limiting factors in plant breeding. They preclude transfer of many desirable traits such as disease, insect and herbicide resistance between species of cultivated or weed plants because of sexual incompatibility. This problem is especially acute in the family Cucurbitaceae, which includes the genera Cucumis (cucumbers [C. sativus], and melons [C. melo]), Citrullus (watermelons) and Cucurbita (squash). Among the cultivated species of the genus Cucumis, successful sexual crosses can be made only between C. sativus and the closely related C. harwickii. Attempts to interbreed cucumbers and other species in this genus failed (Deakin et al., 1971).

The recent advances in genetic engineering seem to be a promising alternative to sexual propagation techniques for improvement of this economically important crop. This is particularly true in the case of tissue culturing where an increase in genetic variation, so-called somaclonal variation, is noted (Larkin and Scowcroft 1981). Further, sterility barriers may be overcome by fusion of protoplasts of sexually incompatible species. However, the lack of a reliable method for regeneration of Cucumis plants by explant tissue culture techniques has prevented significant progress until our recent development of the CTM-1 through CTM-4 series of explant embryoid generation and development media, as reported by us in Trulson, A.J. and Shahin, E.A., In vitro plant regeneration in the genus Cucumis (in press 1986) and covered in our copending application (92/9).

Somatic hybridization permits creation of novel plants by fusion of protoplasts, and/or cytoplasts, derived from somatic tissues of sexually incompatible plants. In this way nuclear as well as cytoplasmic genes may be transferred and combined. Alternatively, in the case of cytoplasmically inherited traits, in which case the transfer of nuclear genes is not desired, one can fuse protoplasts of one species (which is the recipient) with cytoplasts of a different donor species of interest. For instance, transfer of male sterility or herbicide resistance must be done by cytoplast fusion since those characteristics are carried by the organelles such as chloroplasts or mitochondria. In the case of protoplast-cytoplast fusion (herein p/c fusion) only cytoplasmic traits of the donor are transferred and incorporated with the protoplasts of the recipient in a newly created cell. Cytoplasts are protoplasts devoid of nuclei, and may be prepared by centrifugation in a mannitol/sucrose gradient to remove the nucleus, or by rendering the nucleus non-functional by radiation.

An efficient and reliable protocol for tomato protoplast isolation, culture and plant regeneration at plating efficiency of 50% using a series of media TM 1 - 5 has been demonstrated by one of us, Shahin, E.A. (1985). Until this invention, there has been only limited success in regeneration of cucumber plants from protoplasts.

The use of glycine to stabilize <u>Cucumis</u> <u>sativus</u> L. protoplasts is reported by Orczyk and Malepszy (1985). An isolation solution was complemented with .1M glycine in the liberation of leaf protoplasts at 25°C. A hanging drop technique was employed to study the effect of various combinations of two growth substances, an auxin and a cytokinin. An NAA and 2ip combination in an agarose bead culture medium resulted in a plating efficiency of 21%. While cell wall regeneration was induced by combinations of 2,4-D and BAP, or IAA and BAP, only sporadic divisions occurred and multicellular aggregates were not seen. The callus produced using NAA and 2ip was transferred to a medium with 2,4-D and 2ip for plantlet and shoot regeneration by the technique of Malepszy and Nadolska-Orczyk, (1983). Consequently, the yellowish compact callus gave rise to embryoidal structures, which when transferred onto hormone free medium developed into plants.

While tissue culturing may be associated with production and/or expression of genetic variation via somaclonal variation, it does not permit somatic hybridization.

Accordingly, there is a great need for a method of isolating protoplasts from <u>Cucumis</u> sp. plant tissue and fusing them with related strains or sexually incompatible species to develop somatic hybrids, or/and regenerating plants from the liberated protoplasts. This is the key to a successful adaptation of novel breeding methodologies and genetic engineering techniques to agriculture to more quickly and selectively produce improved plants having desirable traits.

The process of the present invention involves production of plantlets by embryogenesis from protoplasts in either "solid" media, such as agar-solidified media of various viscosities, or cell suspension culture where the cells are suspended in a gently agitated liquid medium. For general background see U.S. Patent 4,548,901 (which is directed to a suspension culture process for production of legume plantlets), and references cited therein, particularly G.G. Henshaw et al. (1982).

## THE INVENTION

### Objects

It is among the objects of this invention to provide processes for the <u>in</u> <u>vitro</u> generation (induction) and regeneration of embryoids and plantlets from protoplasts which are capable of developing into mature plants.

It is another object to provide novel media for the induction of embryogenesis and production of embryoids from protoplasts, which embryoids are capable of development to plantlets, and thence into fully mature plants.

It is another object of this invention to provide methods and media for isolation (liberation) of protoplasts from <u>Cucumis</u> sp. plant tissue, for development of cell walls, and for division into protoplast-derived minicalli.

It is another object of this invention to provide methods of continuously producing plant embryoids from protoplasts for encapsulation as artificial seeds.

It is another object of this invention to apply the novel methods and media of this invention to the production of improved plants in the genus <u>Cucumis</u> .

It is another object of this invention to shorten the time for producing genetically engineered and/or hybridized plant stock without having to go through full seed development.

It is another object of this invention to asexually propagate plants without hormonal or chemical induction of flowers.

It is another object of this invention to provide a protoplast culture system which may be used in conjunction with various methods of introducing foreign genes into <u>Cucumis</u> sp.

It is another object of this invention to induce embryoids from protoplasts for frozen storage followed by thawing and continuation of maturation and development into plantlets and/or plants.

It is another object of this invention to provide methods of asexual somatic hybridization using intra and inter species protoplast or protoplast-cytoplast fusions followed by generation of hybrid embryoids and their maturation into hybrid plantlets and plants.

Still other objects of this invention will be evident from the balance of this specification and claims.

Definitions

The terminology used herein is not intended to vary from the terminology used in the field. However, the meaning of some terms used in the field is not necessarily uniform, and the following definitions will be of help in this case:

"Plantlet" refers to a plant sufficiently developed to have a shoot and a root that is asexually reproduced by cell culture.

"Explant" refers to a section or a piece of tissue from any part of a plant for culturing.

"Hormone" refers to a plant growth regulator that affects the growth or differentiation of plants, and is exogenous as used in reference to the various media herein.

"Callus", and its plural "calli" refer to an unorganized group of cells formed in response to a cut, severing or injury of a plant, and herein refers to the unorganized cell growth which may form on explant tissues during culturing or from division of protoplasts which have regenerated cell walls.

"Embryoid" refers to a structure similar in appearance to plant zygotic embryo.

Abbreviations:

NAA = alpha-naphthaleneacetic acid
ZR = Zeatin Riboside
BAP = 6-benzylaminopurine
$GA_3$ = Giberellic acid
2,4-D = 2,4-dichlorophenoxyacetic acid
MS Medium - Murashige and Skoog Medium (Murashige and Skoog, 1962)
CPM = Cucumis Protoplasts Medium
CPE = Cucumis Protoplast Enzyme solution
CPE-G = Cucumis Protoplast Enzyme solution containing glycine
CTM-4 = Cucumis Transformation Medium 4
PET Solution = Tomato pre-enzymatic solution (Shahin, 1985)
TM-1 = Tomato Medium 1 (Shahin 1985)
TM-2 = Tomato Medium 2 (Shahin 1985)

Summary:

We have discovered that a series of three media, called CPM 1-3 media, containing two or more hormones selected from the group comprising NAA, ZR, 2,4-D, BAP, and $GA_3$ are able to induce the development of embryoids from protoplasts of the genus Cucumis in a variety of growth media ranging from tissue culture on solid (agar) substrates to cell suspension, hanging drop or bead techniques. The hormones are present in the CPM-1, -2 and -3 media in the range of from about .2 to about 20 µM as set forth in Table I below:

### Table I
### Hormone Concentration Ranges

| Hormone | Media | $\mu$ M Overall | Preferred |
|---|---|---|---|
| NAA | CPM-1 | .5 - 10 | .5 - 5 |
| ZR | CPM-1 | .2 - 5 | .2 - 2 |
| 2,4-D | CPM-1 | .5 - 10 | .5 - 5 |
| | CPM-2 | 1 - 20 | 1 - 10 |
| | CPM-3 | .5 - 10 | .5 - 5 |
| BAP | CPM-1 | .5 - 10 | .5 - 5 |
| | CPM-2 | .5 - 15 | .5 - 10 |
| | CPM-3 | .5 - 10 | .5 - 5 |
| $GA_3$ | CPM-1 | .5 - 10 | .5 - 5 |

All five hormones are contained in a first, induction culturing medium, CPM-1 Medium, in which cell walls form and the cells undergo division into a multi-cellular colony stage of 10 - 15 cells. CPM-1 Medium is a modified TM-2 (Shahin, 1985) Medium. Thereafter, the multi-cellular colonies are transferred to a first development medium, CPM-2 Medium, containing 2,4-D and BAP, in which they continue to enlarge by cell division into mini-calli. The mini-calli are plated on a second development medium, CPM-3 Medium, which also contains 2,4-D and BAP but at half the concentration of the CPM-2 Medium. Embryoid expression (maturation) occurs in this CPM-3 Medium. Thereafter the fully mature embryoids are transferred to a plantlet developmental medium, CTM-4 Medium, which is hormone free and promotes shoot elongation, rooting and normal plant development, by procedures more fully described in our copending case 92/9.

Both the induction culturing in CPM-1 medium, and the development in CPM-2 and CPM-3 media, occur in the temperature range of from 20 - 30°C, with 25°C ± 2°C being preferred. The induction culturing period is on the order of 5 - 14 days, with 5 - 10 being preferred, in the serially diluted CPM-1 media. The two development culturing steps taken together range from about 13 to less than about 35 days, being less than 7 and preferably 3 - 5 days in the first stage involving CPM-2 media, and less than 28 and preferably 10 - 25 days for the second development stage involving CPM-3 media. The culturing photoperiods range from about 10 hours to continuous light, with 16 hours being preferred. The light intensity should be relatively constant during CPM-1 and CPM-2 culturing in the range of from about 100 to 1,500 lux, with 500 ± 100 lux being preferred. In CPM-3 developmental culturing, the light may be in the range of 4,500 ± 500 lux.

The methods of this invention permit regeneration of plants from protoplasts liberated from any Cucumis sp. tissue be it wild or cultivated, hybrid (somatic or sexual), or genetically variant or transformed. Thus, by the use of the methods and media of this invention it becomes unnecessary to treat gynoecious or androecious plants of the genus Cucumis with external hormones or chemical substances to induce male and/or female flowers. The current practice calls for such induction, followed by pollination and seed production.

By the practice of this invention, field-ready plants can be produced in 60 - 100 days after protoplast liberation (isolation), as compared to six months for production of seed from seed-derived plants. The advantages are particularly significant in hybridization. For example, conventional known techniques involve the steps of development of inbred (parental) lines, of cross pollination, development of F-1 seed, followed by the planting of F-1 seed. The maintenance of the parental lines the making of the F-1 cross is very expensive and time consuming.

However, by the techniques of the present invention involving plant propagation in vitro, hybrid (F-1) plantlets can be reproduced within 12 weeks, with no need to propagate the parental lines and/or to continue making the crosses.

Detailed Description of the Best Mode of Carrying Out the Invention

The following detailed description is by way of example and not by way of limitation of the principles of this invention, and has reference to specific examples in which protoplast isolation followed by embryo generation (induction) and plantlet regeneration within the scope of this invention is described for cucumber (Cucumis sativus L.) by way of example.

1. CPM-1, -2, and -3 Media Composition

The composition of media used in culturing cucumber protoplasts is set forth below in Table 2. CPM-1 contains all five hormones, while CPM-2 and CPM-3 contain sucrose and only two of the hormones, but at different concentrations and other variations. CPM-1 and -2 media are filter-sterilized, medium CPM-3 is solidified with agar (0.7%) and sterilized by autoclaving at 121°C for 15 minutes.

TABLE 2
Media Composition

| Component | mg/l (except as noted) | | |
|---|---|---|---|
| | CPM-1 | CPM-2 | CPM-3 |
| Macronutrients | | | |
| $KH_2PO_4$ | 170 | 170 | 170 |
| $CaCl_2 \cdot 2H_2O$ | 940 | – | 200 |
| $KNO_3$ | 1.5g | 1.5g | 1.5g |
| $NH_4NO_3$ | – | 100 | 200 |
| $MgSO_4 \cdot 7H_2O$ | 370 | 370 | 370 |

## Micronutrients

| | | | |
|---|---|---|---|
| KI | 0.38 | 0.38 | 0.38 |
| $H_3BO_3$ | 6.20 | 6.20 | 6.20 |
| $MnSO_4 \cdot 4H_2O$ | 22.3 | 22.3 | 22.3 |
| $ZnSO_4 \cdot 7H_2O$ | 8.60 | 8.60 | 8.60 |
| $Na_2MoO_4 \cdot 2H_2O$ | 0.25 | 0.25 | 0.25 |
| $CuSO_4 \cdot 5H_2O$ | 0.025 | 0.025 | 0.025 |
| $CoCl_2 \cdot 6H_2O$ | 0.025 | 0.025 | 0.025 |
| $FeSO_4 \cdot 7H_2O$ | 13.90 | 13.90 | 13.90 |
| $Na_2EDTA$ | 18.50 | 18.50 | 18.50 |

## Vitamins

| | | | |
|---|---|---|---|
| Nicotinic acid | 2.50 | 2.50 | 2.50 |
| Thiamine HCl | 10 | 10 | 10 |
| Pyridoxine HCl | 1 | 1 | 1 |
| Folic acid | 0.50 | 0.50 | 0.50 |
| Biotin | 0.05 | 0.05 | 0.05 |
| D-Ca-Pantothenate | 0.50 | 0.50 | 0.50 |
| Choline chloride | 0.10 | 0.10 | 0.10 |
| Glycine | 0.50 | 0.50 | 0.50 |
| Casein Acid Hydrolysate | 150 | 150 | 150 |
| L-Cysteine | 1 | 1 | 1 |
| Malic acid | 10 | 10 | 10 |
| Ascorbic acid | 0.50 | 0.50 | 0.50 |
| Adenine Sulfate | 40 | 40 | 40 |
| Riboflavin | 0.25 | 0.25 | 0.25 |
| Arginine | 10 | - | 10 |
| L-Asparagine | 100 | 250 | 100 |
| L-Glutamine | 100 | 300 | 200 |

## Others

| | | | |
|---|---|---|---|
| Sucrose | - | 10.0g | 10.0g |
| Glucose | 36.00g | 32.5g | 27.0g |
| Mannitol | 4.56g | - | - |
| Xylitol | 3.80g | - | - |
| Sorbitol | 4.56g | - | - |
| M-inositol | 4.60 | 100 | 100 |
| MES | 97.60 | 97.60 | 97.60 |
| Purified agar | - | - | 7.0 |

## Hormones

| | | | |
|---|---|---|---|
| NAA | 0.20 | - | - |
| Zeatin Riboside | 0.25 | - | - |
| 2,4-D | 0.50 | 1.00 | 0.50 |
| BAP | 0.25 | 0.50 | 0.25 |
| $GA_3$ | 0.40 | - | - |

2. Source of Cucumis Tissue For Protoplast Liberation.

Seeds of cucumber cv. Straight Eight (ARCO Seed, Brooks, Oregon) are sterilized in 10% (v/v) Clorox (commercial bleach containing 5.25% sodium hypochlorite) with a drop of Tween 80 (one droplet per 100 ml of the sterilizing solution) for 10 minutes, then rinsed 3× in sterile, distilled water and placed in sterile petri plates (ca 30 per plate) lined with moist Whatman #3 filter paper. To assure uniform and rapid germination of the seeds, plates are kept for 24 - 30 hours at 27°C, in the dark. Germinated seeds (radicle length ca 5mm) are placed aseptically in Magenta boxes (six seeds per box) containing ca 40 ml of TM-1 medium (Shahin, 1985) supplemented with 150 mg/l of Carbenicillin (Sigma), and incubated for four days in a growth chamber, at 21°C night, 26°C day, 14 hour photoperiod (4500 lux). When the seedlings are green, but the cotyledons only partially unfolded, Magenta boxes are placed for 24 hours in the dark, at room temperature.

Example A. Regeneration from Protoplasts

1. Liberation of Protoplasts.

Protoplasts were obtained from cotyledonary tissue of the aseptically grown, five-day-old seedlings of cucumber cv. Straight Eight described above. Protoplast isolation procedure was as follows: The cotyledons from 24 - 30 seedlings were cut asceptically into 10 mm$^2$ pieces and soaked in 50 ml of tomato pre-enzymatic treatment (PET) solution in a 250 ml flask, for 6 - 8 hours at 4°C. PET solution is composed of 1/4$^\times$TM-2 macronutrients (Shahin, 1985), 0.3M sucrose, vitamins as in TM-1 Medium (Shahin, 1985), supplemented with 1.0 mg/1 2,4-D (4.5 $\mu$M), and 0.5 mg/l BAP (2.2 $\mu$M). The pH was adjusted to 5.80 before autoclaving the medium at 121°C for 15 minutes.

Protoplasts were then isolated from cotyledon pieces by replacing the PET solution with 40 ml of filter-sterilized enzyme solution consisting of 0.1% (w/v) Macerozyme (Yakult Biochemicals Co., Ltd., Japan), 0.75% (w/v) Cellulase RS (also Yakult), 0.3M sucrose, TM-2 macronutrients and vitamins (Shahin, 1985), 1.0% polyvinylpyrolidine (PVP-10), 5 M 2[N-Morpholino]-ethane Sulfonic acid (MES) buffer, pH 5.6 (herein CPE solution). The CPE enzyme solution may be supplemented with glycine (Orczyk and Malepszy, 1985) in an amount sufficient to stabilize the protoplasts, e.g. on the order of 1 to 250 mM (herein CPE-G solution). Protoplasts were released after 12 - 14 hours of rotation at 60 rpm in a 26°C water bath shaker. The protoplasts were separated from the undigested tissue by straining through a double layer of sterile cheesecloth, and collected in Babcock bottles (Kimble, series 1000). They were then separated from the enzyme solution by centrifugation at 50G for 10 minutes. The protoplasts were then suspended in tomato rinse solution (0.3M sucrose, 1/2 $^\times$ macronutrients of TM-2, vitamins of TM-2, 0.005M MES buffer, pH 5.8; Shahin 1985), and centrifuged at 50G for 10 minutes.

2. Culturing Protoplasts. Cell Wall Formation in CPM-1 Medium.

One million protoplasts were cultured in 2 ml of the CPM-1 medium, in each of 60 $^\times$ 15 mm plastic petri-dishes. NAA was 1 $\mu$M, ZR .7 $\mu$M, 2,4-D 2.25 $\mu$M, BAP 1.1. $\mu$M and GA$_3$ 1.15 $\mu$M. The petri dishes were sealed with parafilm and incubated at 25°C in diffused light (500 lux), 16 hour photoperiod. Cell wall formation was investigated by Celluofluor (Polysciences, Warrington, PA 18976) staining (Hahne et al., 1983) on the day following protoplast purification.

3. Cell Division in Serially Diluted CPM-1 Medium

During the first week of culture, the osmolarity of the CPM-1 medium was gradually lowered by diluting twice (1:1, v/v) with CPM-1 medium containing 20 g/l of glucose. Multicellular colonies formed after 5 - 10 days of protoplast culture.

4. Minicalli Formation - Induction of Embryoids in CPM-2 Medium (Competent Minicalli Induction Medium)

The colonies formed in 3. were transferred to CPM-2 medium and incubated under the same conditions for 3 - 5 days during which time competent minicalli formed. 2,4-D was 4.5 $\mu$M and BAP was 2.2 $\mu$M.

### 5. Plating of Minicalli - Promotion of Embryoid Maturation (Expression)

The protoplast-derived minicalli were transferred onto semi-solid CPM-3 medium (Embryoid Induction/Expression Medium) in 100 × 20 mm petri-dishes at a density of 200 - 300 minicalli per plate. The 2,4-D was 2.25 μM and BAP was 1.1/μM. The plates were sealed with parafilm and incubated at 25°C, 16 hour photoperiod (4500 lux), for 10 - 25 days, with the resultant formation of glossy, yellowish to yellowish-green competent embryoids, and/or embryoids with visible green cotyledonary structures being discernable.

### 6. Plantlet Development

The embryoids developed in part 5 above were transferred onto CTM-4 medium (Plantlet Development Medium) to promote shoot formation and then handled in the same manner as the plantlets regenerated from the cotyledonary explants. This is described in more detail in our copending case (92/9). CTM-4 medium is set forth in Table 2 below.

TABLE 3
CTM-4 Medium

Mineral Salts

    MS Medium major elements

    MS Medium minor elements

| Organic Constituents: | amount/l |
|---|---|
| Casein hydrolysate | 1.0g |
| Glycine | 2.5mg |
| m-inositol | 100mg |
| Nicotinic acid | 5mg |
| Pyridoxin HCl | 0.5mg |
| Thiamine HCl | 0.5mg |
| Folic acid | 0.5mg |
| Biotin | 0.05mg |
| Sucrose | 30.0g |
| Agar purified (Difco) | 7.0g |

Hormones:

    None

Well developed cucumber plantlets formed approximately 30 days after the last transfer.

### 7. Final Development

The plantlets are placed on an acclimatizing solid agar medium, using medium CTM-4 diluted 50/50 with water, and grown to develop a good root system. They are then potted in soil. The regenerated plants proceed to final development being normal in physiology and morphology, and are fertile, flower, produce fruit and germinable seed.

8. Discussion

Up to three million of viable protoplasts were readily obtained form one gram of cucumber cotyledonary tissue. The protoplasts formed cell walls on the day following their purification. About 5 - 10% of the protoplasts divided on the second day and up to 90% of the remaining protoplasts divided within the next two days. By the end of the first week, many 10 to 15-cell colonies were formed. They continued to enlarge in the CPM-2 medium into mini-calli. After 2 - 3 weeks on the semi-solid CMP-3 medium, 5 - 10% of the mini-calli produced embryoids ranging from globular to early torpedo. About 1% of these embryoids continued to develop into normal, bipolar embryos that could be grown to mature plants. These plants looked normal, flowered, and produced seeds. The remaining embryoids developed into roots with underdeveloped, stubby shoot apices which failed to produce shoots.

In contrast, Coutts and Wood (1977) were only able to induce roots that did not mature into plantlets or plants. While Orczyk and Malepszy (1985) were able to regenerate plants using different media and techniques, they reported low viability of cucumber protoplasts.

Example B. Somatic Hybridization.

Somatic hybridization is accomplished by protoplast-protoplast or protoplast-cytoplast fusion. The protoplasts of different species or cultivars having desired traits should be separately liberated and prepared as in Example A.1. above. Similarly, cytoplasts are prepared by differential centrifugation (in a mannitol/sucrose gradient) to remove the nucleus. They are washed of all salts and suspended in a solution of CPM-1 sugars, at a concentration of approximately 500,000 protoplasts/ml.

Protoplasts of the two different strains should then be combined into a single sample in the CPM-1 sugars medium, and appropriately-sized aliquots (on the order of .2 - .4 ml depending on the fusion chamber size) of the mixture of protoplasts are subjected to constant DC current on the order of 1000 V/cm for a short duration (on the order of 50 sec). This can be preceded by a short (on the order of 1 minute) exposure to low voltage AC (on the order of 150 V/cm) to align the protoplasts in a chain. The DC electric current disrupts the membranes and adjacent protoplasts fuse because of their proximity in the chain. The membrane reseals and fusion into polyploids (e.g. tetraploids for fusion of two protoplasts) occurs. The polyploid protoplasts are then placed in CPM-1 medium for cell wall formation. The procedure for regeneration proceeds as in Example A, steps 2 - 7.

Similarly, protoplast/cytoplast combination (cybridization) will proceed by electrofusion (as described above) before cell wall formation in CPM-1 medium. In this case the ploidy stays the same. Alternatively, three or more species, cultivars, strains or the like, with two or more being protoplasts or cytoplasts may be combined, with correspondingly resultant polyploidy. For example two different protoplasts and a third cytoplast can result in only tetraploidy with cytoplast-carried traits from three different sources. Thus, where herbicide resistance is coded on chloroplasts, the protoplast/cytoplast (hereafter p/c) fusion, or p/p/c or p/c/c fusion (generically $p_x/c_y$ fusion), can transfer that trait to other species, cultivars etc. via this method of somatic hybridization (which includes p/p hybrids and p/c cybrids). Prior to electrofusion, the protoplasts and/or cytoplasts can be genetically engineered using the methods of electroporation or cocultivation.

Alternatively, the p/p and/or p/c fusion can be induced chemically, e.g., by addition of polyethylene glycol (PEG) to the p/p or p/c mixture followed by treatment in a high pH $Ca^{++}$ solution. The addition of PEG results in close adhesion and fusion of the protoplasts and/or protoplasts and cytoplasts. The fusion is completed during the elution of the PEG with the high pH $Ca^{++}$ solution.

Further, the techniques described above can also be used to genetically transform protoplasts by fusing them with liposomes (phospholipid bilayer vesicles) that may contain foreign DNA, or bacterial sphereoplasts, which are bacterial cells devoid of cell walls.

## Example C. Encapsulation - Production of Artificial Seeds.

Since in vitro production of somatic embryoids results in embryoids without protective seed coats, the protoplast-derived embryoids of this invention may be encapsulated, individually or in groups, in capsules or coatings to retard dehydration and preserve them for future "planting". The capsules or coating may be any suitable composition of insoluble material or a slowly soluble gelatine or polymer composition, preferably opaque or semi-opaque, and preferably containing developmental retardant(s) to block precocious germination (further development). The capsules may be empty or contain a medium-hard agar with the appropriate medium therein.

For the capsule embodiment, we prefer to coat or embed mature embryoids in a CTM-4 Medium containing from $10^{-5}$ to $10^{-7}$ M ABA (abscisic acid) hardened with agar. Gelatine capsules can then be filled with a mix of the embryoids and agar/CTM-4/ABA. In the alternative, the agar/CTM-4/ABA-coated embryoids may be over-coated with 2 - 5% polyethylene oxide solution, and dried on teflon paper sheets or in suitably-sized screen interstices.

In the alternative, the bare embryoids can be directly drop-coated with polyethylene oxide (.5 - 10% solutions) to form a continuous coating thereon when dry. The ABA may be added to the polyethylene oxide solution.

In addition, the mature protoplast-derived embryoids developed in the CPM-3 solution may be subjected to a retardation pre-treatment in an ABA solution for from 3 - 7 days before the encapsulation.

The thus-encapsulated protoplast-derived embryoids may be "grown" as follows. In the case of the insoluble capsules, the capsules may be shipped and opened on site with the embryoids (coated or bare) implanted on plates containing CTM-4 Medium from which the development of rooted plantlets/plants are then transplanted to fields. In the case of the soluble capsules and polyethylene oxide-coated embryoids, they may be sown in agar containing CTM-4 Medium, or sown directly into rooting medium such as "Plant-Lite" with appropriate nutrients. Upon contacting the moistened agar or the wet rooting medium, the capsules and/or coating(s) will dissolve, the ABA will be diluted to ineffectiveness, and the embryoids will be released to continue development into plantlets/plants as above-described.

## Example D. Regeneration after Freeze Storage.

One or more of the protoplast sources, protoplasts therefrom, mini-calli, or embryoids (at various stages including in encapsulated form), may be stored frozen (dry ice, or, preferably, liquid nitrogen) for extended periods, then thawed and regenerated following the steps and media set forth above. The protoplast-derived embryoids, etc. may be coated with agar-hardened CTM-4 Medium (with or without retardants) prior to freezing. This technique of freezing competent embryoids, etc. provides a convenient genetic information storage method. Any of the well-known, conventional freezing, storage and thawing techniques for tissue, cells (e.g. sperm banks), and cell cultures (e.g. ATCC type cultures) may be employed in combination with the novel media and methods herein.

It should be understood that various modifications within the scope of this invention can be made by one of ordinary skill in the art without departing from the spirit thereof. For example, the positive response of Cucumis sp. protoplasts to regeneration by the media and methods of this invention render them useful in breeding. One important application is in the production of gynoecious populations to replace the current expensive treatment with silver nitrate. Another application of this invention is to somatic hybridization and/or somaclonal variation as a valuable source of diversity in plant material used in breeding. The protoplast fusion and regeneration procedures herein may be used for selection of individuals resistant to pathogens, toxic metals, pesticides and herbicides. The latter is of particular importance because cucumbers are very sensitive to herbicides.

The application of genetic engineering in cucumber and muskmelon breeding is of special value. These two species are sexually incompatible. Conventional crosses and transfer of many desirable traits like disease resistance heretofore have not been possible (Deakin et al., 1971). Such barriers are now removed with the development of capability of gene transfer through somatic hybridization disclosed herein, and plant transformation disclosed in our copending case 92/12, in which gene transfer is accomplished transforming roots using A. rhizogenes.

In addition to the ability to transfer horticulturally desirable genes into cucumber or muskmelon, genetic engineering procedures are particularly valuable in introduction of marker genes such as resistance to kanamycin or chloramphenicol. These markers facilitate somatic hybridization via protoplast fusion, thus removing the sterility barriers in the genus Cucumis.

# 0 265 100

We therefore wish our invention to be defined by the scope of the appended claims as broadly as the prior art will permit, and in view of this specification if need be.

## REFERENCES

1. Coutts, R.H.A, and Wood, K.R. (1977): Improved isolation and culture methods for cucumber mesophyll protoplasts. Plant Sci Let 9:45-51.

2. Deakin, J.R., Bohn, G.W., and Whitaker, T.W. (1971): Interspecific hybridization in Cucumis. Econ Bot 25:195-211.

3. Hahne, G., Herth, W., and Hoffmann, F. (1983): Wall formation and cell division in fluorescence labelled plant protoplasts. Protoplasma 115:217-221.

4. Larkin, P.J., and Scowcroft, W.R. (1981): Somaclonal variation - a novel source of variability from cell cultures for plant improvement. Theor Appl Genet 60:197-214.

5. Malepszy, S., and Nadolska-Orczyk, A. (1983): In vitro culture of Cucumis sativus L. Regeneration of plantlets from callus formed by leaf explants. Z Pflanzenphysiol Bd 111:273-276.

6. Murashige, T., and Skoog, F. (1962): A revised medium for rapid growth and bioassays with tobacco tissue cultures. Physiol Plant 15:473-497.

7. Orczyk, W., and Malepszy, S. (1985): In vitro culture of Cucumis sativus L. V. Stabilizing effect of glycine on leaf protoplasts. Plant Cell Reports 4:269-273.

8. Shahin, E.A. (1985): Totipotency of tomato protoplasts. Theor Appl Gent 69:235-240.

9. Henshaw, G.G., et al. (1982): Morphogenic Studies in Plant Tissue Cultures. In: Differentiation in Vitro. M.M. Yeoman and D.E.S. Truman, eds., Cambridge Press.

## Claims

1. Method of regeneration of competent tissue from Cucumis sp. protoplasts comprising the steps of:

a) isolating protoplasts from Cucumis sp. plant tissue;

b) induction culturing said protoplasts in a first, cell development medium containing an effective amount of exogenous hormones NAA, ZR, 2,4-D, BAP and $GA_3$ for a time and under conditions sufficient to induce new cell wall formation and promote cell division to form multi-cellular colonies;

c) development culturing said multi-cellular colonies in at least one additional development medium containing an effective amount of exogenous hormones BAP and 2,4-D for a time and under conditions sufficient to induce development of competent tissue capable of completing development into plantlets, said competent tissue being selected from competent mini-calli and embryoids;

d) optionally culturing the mature embryoids in a plantlet regeneration medium essentially free of said hormones NAA, BAP, and 2,4-D for a time period sufficient to develop a plantlet; and

e) optionally storing said mini-calli or embryoids in a frozen condition.

2. The method of claim 1 wherein:

said induction and development culturing steps are maintained in a temperature range of from about 20-30°C, and for a photoperiod of from about 10 hours to continuous light in an intensity range of from about 100 to about 5,000 lux, and wherein

said hormones are present in said first medium in the range of from about .2 to about 5 $\mu$M and in said second media in the range of from about .5 to about 10 $\mu$M.

3. The method of claim 2 wherein said induction medium is CPM-1 medium and said development culture medium is selected from CPM-2 medium, CPM-3 medium, and a sequence of CPM-2 medium followed by CPM-3 medium.

4. The method of claim 1 wherein:

a) said development culturing occurs serially in at least two media, a first, mini-calli development medium, and a second, embryoid expression medium;

b) said first development culturing is continued for a time sufficient to develop competent mini-calli; and which includes the step of:

c) removing said mini-calli and transferring them to said second, embryoid medium for culturing an additional development period of time sufficient to produce competent embryoids.

5. The method of claim 4 wherein said first development medium is CPM-2, and said second development medium is CPM-3.

6. The method of claim 1 wherein during said step (d) of plantlet regeneration, secondary or tertiary embryoids are formed; and wherein step (d) further includes the step of transferring and culturing said secondary or tertiary embroyids in said plantlet regeneration medium to substantially continuously produce competent embryoids.

7. Competent Cucumis sp. mini-calli or embryoids produced by the process of claim 1

8. Competent Cucumis sp. mini-calli or embryoids of claim 7 encapsulated as artificial seeds.

9. Cucumis sp. plantlets produced by the process of claim 1.

10. A method of culturing mini-calli or embryoids which comprises

thawing mini-calli or embryoids which have been prepared according to claim 1 and frozen, and culturing said thawed mini-calli or embryoids in a medium to promote growth and regeneration.

11. Protoplast culture medium selected from the group consisting of:

a) TM-2 medium modified to contain exogenous hormones NAA, ZR, BAP, GA$_3$ and 2,4-D, said hormones being present in said medium in a concentration in the range of from about .2 to about 20 μM; and

b) TM-2 medium modified to contain exogenous hormones 2,4-D and BAP, said hormones being present in said medium in a concentration in the range of from about .2 to about 20 μM.

12. The method of claim 1 which includes, prior to said induction culturing, the step of:

a) fusing one or more cellular-derived material(s) selected from one or more protoplast(s), cytoplast-(s), liposome(s) and mixtures thereof, from one Cucumis sp. recipient plant, with one or more cellular-derived material selected from one or more protoplast(s), sphereoplast(s), liposome(s), cytoplasts and mixtures thereof from another, donor organism to produce a viable genetically altered Cucumis sp. mini-calli or embryoid.

13. Competent genetically altered mini-calli or embryoids produced by the process of claim 12 and plantlets regenerated therefrom.

14. Enzyme solution for use in a process of liberation of protoplasts from plant tissue comprising:

a) a sterile aqueous mixture of macerozyme, cellulase RS, sucrose, TM-2 macronutrients, TM-2 vitamins, PVP-10 and MES, said mixture being adjusted to pH 5.6.